# EUROPÄISCHE PATENTSCHRIFT

(11) **EP 2 440 538 B1**
(45) Veröffentlichungstag und Bekanntmachung des Hinweises auf die Patenterteilung: **16.07.2014**
(21) Anmeldenummer: 10724836.1
(22) Anmeldetag: 09.06.2010
(51) Int. Cl.: C07D 301/10

(54) **VERWENDUNG STRUKTURIERTER KATALYSATORBETTEN ZUR HERSTELLUNG VON ETHYLENOXID**
USE OF STRUCTURED CATALYST BEDS TO PRODUCE ETHYLENE OXIDE
UTILISATION DE LITS DE CATALYSEUR STRUCTURÉS POUR LA PRODUCTION D'OXYDE D'ÉTHYLÈNE

(30) Priorität: 09.06.2009 EP 09162254
(43) Veröffentlichungstag der Anmeldung: 18.04.2012
(73) Patentinhaber: BASF SE, 67056 Ludwigshafen (DE)
(72) Erfinder: SEEBER, Georg, 67245 Lambsheim (DE); MÄURER, Torsten, 67245 Lambsheim (DE); THEIS, Gerhard, 67133 Maxdorf (DE); KÖFFER, Dieter, 69488 Birkenau (DE); ROSOWSKI, Frank, 68239 Mannheim (DE)
(86) Internationale Anmeldenummer: PCT/EP2010/058065
(87) Internationale Veröffentlichungsnummer: WO 2010/142714

(56) Entgegenhaltungen:
- EP-A1- 0 428 845
- EP-A1- 0 557 833

## Beschreibung

Die vorliegende Erfindung betrifft ein Verfahren zur Herstellung von Ethylenoxid durch die Umsetzung von Ethylen mit Sauerstoff in Gegenwart mindestens eines silberhaltigen Katalysators, wobei die Umsetzung in einem Reaktor stattfindet, der eine Katalysatorschüttung mit mindestens zwei Schichten (i) und (ii) aufweist, und der Silbergehalt des Katalysators in der Schicht (i) geringer ist als der Silbergehalt des Katalysators in der Schicht (ii), wobei das Reaktionsgemisch mit der Schicht (i) in Kontakt kommt, bevor es mit der Schicht (ii) in Kontakt kommt.

Ethylenoxid, eine wichtige Grundchemikalie, wird industriell durch die Oxidation von Ethylen mit Sauerstoff in Gegenwart silberhaltiger Katalysatoren hergestellt. Üblicherweise werden hierzu Trägerkatalysatoren verwendet, auf die das katalytisch aktive, metallische Silber mittels eines geeigneten Verfahrens aufgebracht worden ist.

Üblicherweise wird bei der Herstellung von Ethylenoxid ein geträgerter Silberkatalysator (Ag auf Al₂O₃) eingesetzt, der eine Silberkonzentration zwischen 15 - 30 Gew.% Silber aufweist. Dabei kann der Katalysator neben Silber auch weitere Aktivmetalle aufweisen.

Aus dem Stand der Technik sind Verfahren bekannt, die strukturierte Katalysatorbetten verwenden, die Gradienten von z.B. Promotoren aufweisen und unterschiedlich aktive bzw. selektive Katalysatorschichten enthalten.

In JP-A 56005471 wird beispielsweise ein Verfahren zur Herstellung von Ethylenoxid beschrieben, in dem alkalimetallhaltige Silberkatalysatoren verwendet werden, die entlang der Länge der Katalysatorschüttung mit unterschiedlichen Mengen an Alkalimetallen beladen sind, derart, dass entlang der Schüttung ein Alkalimetallkonzentrationsgradient vorliegt. Am oberen Ende der Katalysatorschüttung, d.h. an der Eintrittstelle des Reaktionsgases, soll nach dieser Schrift der Alkalimetallgehalt des Silberkatalysators niedriger sein als am unteren Ende der Katalysatorschüttung, also am Austritt des Reaktionsgases. Da die Alkalimetalldotierung des Silberkatalysators eine Abnahme der Aktivität und eine Erhöhung der Selektivität des Katalysators für die Bildung von Ethylenoxid zur Folge hat, kommt das Reaktionsgasgemisch beim Passieren des Reaktors mit zunehmend selektiverem und abnehmend aktiverem Silberkatalysator in Kontakt.

EP 0 557 833 A1 offenbart eine Kombination von hochselektiven und hochaktiven Katalysatoren in einer strukturierten Katalysatorschüttung. Es wird ein Verfahren zur Herstellung von Ethylenoxid durch die Oxidation von Ethylen mit Sauerstoff in Gegenwart silber- und promotorhaltiger Katalysatoren beschrieben, wobei mindestens zwei Silberkatalysatoren unterschiedlicher Selektivität und Aktivität in einer kombinierten Katalysatorschüttung verwendet werden.

Aus EP 0 428 845 A1 ist ein Katalysator bekannt, der einen Silber-Gradienten innerhalb eines einzelnen Katalysatorformkörpers aufweist.

WO 2004/078711 offenbart ein Verfahren zur Herstellung von Ethylenoxid, das bei reduzierten CO2-Konzentrationen betrieben wird. Dabei werden hochselektive und hochaktive strukturierten Katalysatorbetten eingesetzt.

In der wissenschaftlichen Veröffentlichung "Oxidation of ethylene to ethylene oxide: catalyst deactivation in an industrial run", Montrasi et al., Applied Catalysis (1983), 5 (3), 359-369, wird eine Untersuchung zur Deaktivierung industrieller EO-Katalysatoren entlang des Reaktorrohres beschrieben.

Bei den aus dem Stand der Technik bekannten Verfahren nimmt während der gesamten Laufzeit des Katalysators im Produktionsbetrieb die Aktivität des Katalysators zunehmend ab. Nach einer Optimierungsphase erreicht der Katalysator seine maximale / optimale Selektivität, welche im weiteren Verlauf des Betriebs dann wieder abnimmt. Zur Beibehaltung einer bestimmten Produktionsmenge (work rate), wird daher üblicherweise die Temperatur im Produktionsbetrieb zunehmend erhöht. Nachteil dieser Fahrweise ist die notwendige, stetige Temperaturerhöhung zur Erhaltung der Produktionsmenge, um das stetige Abnehmen der Selektivität des Katalysators nach Erreichen des Selektivitätsmaximums auszugleichen.

Eine Bestrebung der Katalysatorforschung ist es, die Betriebstemperatur des Katalysators und damit einen Einflussfaktor auf die Deaktivierung über einen möglichst langen Bereich so gering zu halten, dass der Katalysator über einen möglichst langen Zeitraum höchstmögliche Selektivitäten erreicht.

Ausgehend von diesem Stand der Technik lag eine der vorliegenden Erfindung zugrunde liegende Aufgabe darin, ein Verfahren für die großtechnische Herstellung von Ethylenoxid bereitzustellen, das eine hohe Langzeitstabilität des Katalysators aufweist.

Erfindungsgemäß wird diese Aufgabe gelöst durch ein Verfahren zur Herstellung von Ethylenoxid durch die Umsetzung von Ethylen mit Sauerstoff in Gegenwart mindestens eines silberhaltigen Katalysators, wobei die Umsetzung in einem Reaktor stattfindet, der eine Katalysatorschüttung mit mindestens zwei Schichten (i) und (ii) aufweist, und der Silbergehalt des Katalysators in der Schicht (i) geringer ist als der Silbergehalt des Katalysators in der Schicht (ii), wobei das Reaktionsgemisch mit der Schicht (i) in Kontakt kommt, bevor es mit der Schicht (ii) in Kontakt kommt.

Erfindungsgemäß findet die Umsetzung von Ethylen mit Sauerstoff in Gegenwart mindestens eines silberhaltigen Katalysators in einem Reaktor statt, der eine Katalysatorschüttung mit mindestens zwei Schichten (i) und (ii) aufweist. Erfindungsgemäß enthalten die Schichten (i) und (ii) einen silberhaltigen Katalysator. Dabei ist der Silbergehalt des Katalysators in der Schicht (i) geringer ist als der Silbergehalt des Katalysators in der Schicht (ii).

Erfindungsgemäß kann die Katalysatorschüttung auch weitere Schichten (iii), (iv), (v), (vi) usw. aufweisen, die einen silberhaltigen Katalysator enthalten. Dabei ist vorzugsweise der Silbergehalt des Katalysators in einer Schicht (x) jeweils höher als der Silbergehalt des Katalysators in der Schicht (x-1). Vorzugsweise weist die Katalysatorschüttung die Schichten (i) und (ii) auf.

Im Rahmen der vorliegenden Erfindung sind die einzelnen Schichten der Katalysatorschüttung im Reaktor so angeordnet, dass sich die Schicht (i) näher am Reaktoreingang befindet als die Schicht (ii), die sich wiederum näher am Reaktoreingang befindet als die Schicht (iii), usw.. Entsprechend wird die Schicht (i) bei der Umsetzung vor der Schicht (ii) durchströmt und diese wiederum vor der Schicht (iii), usw. Demnach wird also eine Schicht (x-1) jeweils vor der Schicht (x) durchströmt.

Es wurde gefunden, dass es bei Verwendung einer mehrlagigen Schüttung eines silberhaltigen Katalysators mit zunehmender Silberkonzentration des Katalysators (Silbergradient) entlang der Katalysatorschüttung (in Fliessrichtung des Gasstroms) möglich ist, die Aktivität der strukturierten Katalysatorschüttung im Vergleich mit den 100% reinen Katalysatoren zu erhöhen, bzw. zu stabilisieren. Unter einem Silbergradienten wird dabei im Rahmen der vorliegenden Erfindung eine kontinuierliche oder diskontinuierliche Änderung der Silberkonzentration verstanden.

Kombiniert man zwei oder mehrere Katalysatoren in einem strukturierten Katalysatorbett, so erzielt man eine Verbesserung, wenn die Konzentration an Silber entlang des Gasstromes im Reaktor zunimmt. Für eine Katalysatorschüttung umfassend die Schichten (i) und (ii) ist dabei die Selektivität proportional zu dem in der Schicht (i) enthaltenen Katalysator mit geringerem Silbergehalt, wobei die Aktivität überraschenderweise durch den in Schicht (ii) der Katalysatorschüttung enthaltenen Katalysator bestimmt wird. So sind abhängig vom eingesetzten Katalysator beispielsweise bereits 50 % der Katalysatorschüttung von Schicht (ii) ausreichend, um die gleiche, hohe Aktivität der 100% reinen Katalysatorsschüttung enthaltend denselben Katalysator zu erreichen.

Durch den Anteil an Schicht (i) ergibt sich ebenfalls eine bessere Selektivität im Vergleich zur 100% reinen Katalysatorschüttung aus Schicht (ii). Daraus ergibt sich für das erfindungsgemäße Verfahren eine deutliche Aktivitätssteigerung durch den Einsatz strukturierter Katalysatorschüttungen.

Selbstverständlich können auch unterschiedliche Mischungen von Silberkatalysatoren erfindungsgemäß in kombinierten Katalysatorschüttungen verwendet werden. Die erfindungsgemäß kombinierten Schüttungen der Silberkatalysatoren können beispielsweise so erzeugt werden, dass man im einfachsten Fall einfach zwei oder mehr Lagen der unterschiedlichen Katalysatoren in einem Reaktionsrohr übereinander schichtet.

Erfindungsgemäß kommt bei der Umsetzung das Reaktionsgemisch somit mit der Schicht (i) und dem darin enthaltnen Katalysator in Kontakt, bevor es mit Schicht (ii) und dem darin enthaltenen Katalysator in Kontakt kommt.

In dem erfindungsgemäßen Verfahren zur Herstellung von Ethylenoxid durch die Umsetzung von Ethylen mit Sauerstoff in Gegenwart mindestens eines silberhaltigen Katalysators wie zuvor beschrieben kommt das Reaktionsgemisch mit der Schicht (i) in Kontakt, bevor es mit der Schicht (ii) in Kontakt kommt.

Im Rahmen der vorliegenden Erfindung ist es bevorzugt, dass jede der Schichten (i) und (ii) der Katalysatorschüttung jeweils mindestens 10% der gesamten Katalysatorschüttung ausmacht, besonders bevorzugt jeweils mindestens 20% der gesamten Katalysatorschüttung, insbesondere jeweils mindestens 30% der gesamten Katalysatorschüttung.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei die Schicht (i) und die Schicht (ii) jeweils mindestens 10% der gesamten Katalysatorschüttung ausmachen.

Dabei können die Schichten (i) und (ii) im Rahmen der vorliegenden Erfindung annähernd gleiche Anteile an der gesamten Katalysatorschüttung ausmachen. Es ist aber ebenso möglich, dass die Schichten (i) und (ii) unterschiedliche Anteile an der gesamten Katalysatorschüttung ausmachen.

Beispielsweise kann im Rahmen einer Ausführungsform der vorliegenden Erfindung die Schicht (i) von 40 bis 70 % der gesamten Katalysatorschüttung ausmachen, vorzugsweise von 45 bis 65 % der gesamten Katalysatorschüttung, insbesondere vom von 50 bis 60 % der gesamten Katalysatorschüttung.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, die Schicht (i) von 40 bis 70 % der gesamten Katalysatorschüttung ausmacht.

Im Rahmen einer weiteren Ausführungsform der vorliegenden Erfindung kann die Schicht (ii) beispielsweise von 20 bis 60 % der gesamten Katalysatorschüttung ausmachen, vorzugsweise von 25 bis 50 % der gesamten Katalysatorschüttung, insbesondere vom von 30 bis 40 % der gesamten Katalysatorschüttung.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei die Schicht (ii) von 20 bis 60 % der gesamten Katalysatorschüttung ausmacht.

Neben den Schichten (i), (ii), (iii), usw. wie oben beschrieben, kann die Katalysatorschüttung weitere Schichten aufweisen. Diese Schichten können einen silberhaltigen Katalysator enthalten. Ebenso ist es jedoch möglich, dass die Katalysatorschüttung weitere Schichten aufweist, die keinen silberhaltigen Katalysator enthalten, also beispielsweise nur ein inertes Material.

Gemäß einer Ausführungsform der vorliegenden Erfindung weist die Katalysatorschüttung neben den Schichten (i), (ii), (iii) usw., vorzugsweise neben den Schichten (i) und (ii), eine vorgelagerte Schicht (a) enthaltend einen silberhaltigen Katalysator auf, wobei diese Schicht näher am Reaktoreingang liegt als die Schicht (i) und der Silbergehalt des Katalysators in der Schicht (a) höher ist als der Silbergehalt des Katalysators in der Schicht (i).

Demgemäß kommt die Schicht (a) und der darin enthaltene Katalysator bei der Umsetzung mit dem Reaktionsgemisch in Kontakt, bevor das Reaktionsgemisch mit der Schicht (i) und dem darin enthaltenen Katalysator in Kontakt kommt.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei die Katalysatorschüttung eine weitere Schicht (a) aufweist, mit der das Reaktionsgemisch vor den Schichten (i) und (ii) in Kontakt kommt, und der Silbergehalt des Katalysators in der Schicht (a) höher ist als der Silbergehalt des Katalysators in der Schicht (i).

Diese vorgelagerte Schicht (a) kann als "Schutzlage" dienen, um die späteren Schichten, insbesondere die Schicht (i) zu schützen, da die Deaktivierung im Betrieb einer EO-Produktionsanlage insbesondere im vorderen Gaseingangsbereich des Reaktors stattfindet.

Die Schicht (a) macht dabei erfindungsgemäß vorzugsweise höchstens 10% der gesamten Katalysatorschüttung aus, insbesondere höchstens 8% der gesamten Katalysatorschüttung, besonders bevorzugt höchstens 6% der gesamten Katalysatorschüttung.

Vorzugesweise weist die Katalysatorschüttung im Rahmen der vorliegenden Erfindung neben den Schichten (a), (i) und (ii) keine weiteren Schichten auf, die einen silberhaltigen Katalysator aufweisen. Die Katalysatorschüttung kann jedoch im Rahmen dieser Ausführungsform weitere Schichten umfassen, die keinen silberhaltigen Katalysator enthalten.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei die Schicht (a) höchstens 10% der gesamten Katalysatorschüttung ausmacht.

Erfindungsgemäß ist es bevorzugt, dass der Silbergehalt des Katalysators in den einzelnen Schichten sich über die Laufzeit des Verfahrens nicht verändert, d.h. dass es vorzugsweise bei der Alterung des Katalysators nicht zu einer Veränderung des Silbergehalts kommt.

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei der Gradient des Silbergehalts des Katalysators in den Schichten der Katalysatorschüttung über die gesamte Laufzeit des Verfahrens vorliegt.

Erfindungsgemäß unterscheidet sich der in den einzelnen Schichten der Katalysatorschüttung enthalte Katalysator durch den Silbergehalt. Dabei ist es im Rahmen der vorliegenden Erfindung möglich, dass der Silbergehalt des Katalysators in einer Schicht (x-1) um 5 bis 15 Gew.-% geringer ist als der Silbergehalt des Katalysators in der Schicht (x). Bevorzugt ist daher der Silbergehalt des Katalysators in der Schicht (i) um 5 bis 15 Gew.-% geringer ist als der Silbergehalt des Katalysators in der Schicht (ii), insbesondere ist Silbergehalt des Katalysators in der Schicht (i) um 8 bis 12 Gew.-% geringer ist als der Silbergehalt des Katalysators in der Schicht (ii), besonders bevorzugt ist Silbergehalt des Katalysators in der Schicht (i) um 9 bis 11 Gew.-% geringer ist als der Silbergehalt des Katalysators in der Schicht (ii).

Daher betrifft die vorliegende Erfindung gemäß einer weitere Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei der Silbergehalt des Katalysators in der Schicht (i) um 5 bis 15 Gew.-% geringer ist als der Silbergehalt des Katalysators in der Schicht (ii).

Beispielsweise weist der in Schicht (i) der Katalysatorschüttung enthaltene Katalysator einen Silbergehalt von 10 bis 20 Gew.-% auf, vorzugsweise von 12 bis 18 Gew.-%, insbesondere von 13 Gew.-%, 14 Gew.-%, 15 Gew.-%, 16 Gew.-% oder 17 Gew.-%.

Der in Schicht (ii) der Katalysatorschüttung enthaltene Katalysator weist beispielsweise einen Silbergehalt von 20 bis 40 Gew.-% auf, vorzugsweise von 22 bis 30 Gew.-%, insbesondere von 23 Gew.-%, 24 Gew.-%, 25 Gew.-%, 26 Gew.-%, 27 Gew.-%, 28 Gew.-% oder 29 Gew.-%.

Erfindungsgemäß kann der in Schicht (a) enthaltene Katalysator beispielsweise denselben Silbergehalt aufweisen wie der in Schicht (ii) enthaltene Katalysator. Demgemäß weist der in Schicht (ii) der Katalysatorschüttung enthaltene Katalysator beispielsweise einen Silbergehalt von 20 bis 40 Gew.-% auf, vorzugsweise von 22 bis 30 Gew.-%, insbesondere von 23 Gew.-%, 24 Gew.-%, 25 Gew.-%, 26 Gew.-%, 27 Gew.-%, 28 Gew.-% oder 29 Gew.-%.

Der in den einzelnen Schichten der Katalysatorschüttung enthaltene Katalysator kann erfindungsgemäß neben Silber weitere Aktivmetalle und Promotoren aufweisen.

Als Silberkatalysatoren können im erfindungsgemäßen Verfahren alle zur Herstellung von Ethylenoxid aus Ethylen und Sauerstoff geeigneten silberhaltigen Trägerkatalysatoren verwendet werden. Als Trägermaterial kann prinzipiell jeder poröse Werkstoff dienen, der unter den Bedingungen der Ethylenoxidsynthese stabil ist, beispielsweise Aktivkohle, Aluminiumoxide, Titan-, Zirkonium- oder Siliciumdioxide oder andere keramische Massen.

Die geometrische Form der Trägerpartikel ist im allgemeinen von untergeordneter Bedeutung, zweckmäßigerweise sollten die Trägerpartikel aber Formen haben, die eine ungehinderte Diffusion der Reaktionsgase an einen möglichst großen Teil der mit den katalytisch aktiven, gegebenenfalls mit Zusatzstoffen dotierten Silberpartikeln belegten äußeren und inneren Oberfläche der Trägerpartikel ermöglicht.

Die katalytisch aktiven Bestandteile der Silberkatalysatoren wie sie im erfindungsgemäßen Verfahren angewandt werden, also Silber und gegebenenfalls zugesetzte Dotierstoffe, können mit allen Tränk- und Abscheideverfahren des Standes der Technik zur Herstellung von Silberkatalysatoren für die Herstellung von Ethylenoxid auf das Trägermaterial aufgebracht werden, wobei diese Verfahren eine oder mehrere Tränkund Kalzinierungsstufen umfassen können. Beispielhaft seien die Herstellverfahren für Silberkatalysatoren genannt, wie sie in DE-A 23 00 512, DE-A 25 21 906, EP-A 14 457, EP-A 85 237, EP-A 384 312, DE-A 24 54 972, DE-A 33 21 895, EP-A 229 465, DE-A 31 50 205, EP-A 172 565 und EP-A 357 293 offenbart sind. Zur Dotierung der im erfindungsgemäßen Verfahren einsetzbaren Silberkatalysatoren mit Zusatzstoffen, die beispielsweise die Aktivität, Selektivität und Standzeit der Silberkatalysatoren beeinflussen, so genannten Promotoren, gibt es prinzipiell keine Einschränkungen, d.h. es können sämtliche Promotoren des Standes der Technik zur Dotierung der Silberkatalysatoren verwendet werden. Als solche Promotoren seien insbesondere Alkalimetall- und Erdalkalimetallhydroxide oder -salze sowie die Verbindungen der Elemente der 6. und 7. Nebengruppe des Periodensystems (Notation gemäß IUPAC-Vorschlag von 1985), insbesondere Verbindungen der Elemente Wolfram, Molybdän und/oder Rhenium zu nennen.

Für die Anionen der Salze der Promotoren gibt es ebenfalls keine Beschränkungen, beispielsweise können alle Halogenide, insbesondere Fluorid, Chlorid, Carboxylate, Nitrat, schwefelhaltige Anionen, wie Sulfat oder Sulfid, Phosphate, Cyanid, Hydroxid, Carbonate oder Anionen von Heteropolysäuren, insbesondere von Heteropolysäuren der Elemente der 6. und 7. Nebengruppe des Periodensystems, besonders bevorzugt Anionen von Heteropolysäuren des Wolframs, Molybdäns und/oder Rheniums, Anionen dieser Salze sein.

Beispielhaft für die mit Promotoren dotierten Silberkatalysatoren, die im erfindungsgemäßen Verfahren eingesetzt werden können, seien die Silberkatalysatoren von DE-A 23 00 512, DE-A 25 21 906, EP-A 14 457, DE-A 24 54 972, EP-A 172 565, EP-A 357 293, EP-A 266 015, EP-A 11 356, EP A 85 237, DE-A 25 60 684 und DE-A 27 53 359 genannt.

Bevorzugt enthält der Katalysator neben Silber als Promotor Rhenium und mindestens ein weiteres Metall. Daher betrifft die vorliegende Erfindung gemäß einer weiteren Ausführungsform ein Verfahren zur Herstellung von Ethylenoxid durch die Umsetzung von Ethylen mit Sauerstoff in Gegenwart mindestens eines silberhaltigen Katalysators wie zuvor beschrieben, wobei mindestens einer der in den Schichten eingesetzten Katalysatoren Rhenium enthält.

Erfindungsgemäß ist es auch möglich, dass mehrere oder alle der in den Schichten eingesetzten Katalysatoren Rhenium enthalten.

Die in den im vorangegangenen Absatz beispielhaft genannten Schriften offenbarten Silberkatalysatoren mit den darin angegebenen Gehalten an Silber und Promotoren, hergestellt nach den darin angegebenen Imprägnier-, Trocknungs-, Silberzersetzungsund Calcinierverfahren, können allesamt mit gutem Erfolg im erfindungsgemäßen Verfahren eingesetzt werden. Für den Erfolg des erfindungsgemäßen Verfahrens ist somit nicht die Art der jeweils eingesetzten Katalysatoren kritisch, sondern allein die Maßnahme, dass kombinierte Schüttungen von Katalysatoren, die sich bezüglich des Silbergehalts unterscheiden, verwendet werden. Allein durch diese Maßnahme können die vorher erwähnten Vorteile, z.B. die Verlängerung der Standzeit der betreffenden Silberkatalysatoren, erzielt werden.

Mit Hilfe der erfindungsgemäßen kombinierten Katalysatorschüttungen der Silberkatalysatoren kann Ethylenoxid nach an sich herkömmlichen Methoden durch die Direktoxidation von Ethylen mit Sauerstoff erzeugt werden. Dazu können alle in den Ethylenoxidherstellungsverfahren des Standes der Technik anwendbaren Reaktoren verwendet werden, beispielsweise die üblicherweise industriell eingesetzten außengekühlten Rohrbündelreaktoren (vgl. Ullmann's Encyclopedia of Industrial Chemistry; 5th Ed.; Vol. A10; S. 117-135, 123-125; VCH Verlagsgesellschaft; Weinheim 1987) als auch Reaktoren mit loser Katalysatorschüttung und Kühlrohren, beispielsweise die Reaktoren gemäß DE-A 34 14 717, EP-A 82 609 und EP-A 339 748. Zur Erzeugung der kombinierten Katalysatorschüttung werden die unterschiedlichen Katalysatoren üblicherweise nacheinander, in der gewünschten Reihenfolge, in den betreffenden Reaktor eingefüllt.

Zur Herstellung von Ethylenoxid aus Ethylen und Sauerstoff mit Hilfe der erfindungsgemäßen Katalysatorschüttungen kann unter herkömmlichen Reaktionsbedingungen, wie sie beispielsweise in DE-A 25 21 906, EP-A 14 457, DE-A 23 00 512, EP-A 172 565, DE-A 24 54 972, EP-A 357 293, EP-A 266 015, EP-A 85 237, EP-A 82 609 und EP-A 339 748 beschrieben sind, gearbeitet werden. Dem Ethylen und molekularen Sauerstoff enthaltenden Reaktionsgas können dabei zusätzlich noch Inertgase wie Stickstoff oder sich unter den Reaktionsbedingungen inert verhaltende Gase, wie Wasserdampf, Methan sowie gegebenenfalls Reaktionsmoderatoren (Inhibitoren), beispielsweise halogenierte Kohlenwasserstoffe, wie Ethylchlorid, Vinylchlorid oder 1,2-Dichlorethan, zugemischt werden. Zweckmäßigerweise liegt der Sauerstoffgehalt des Reaktionsgases in einem Bereich, in dem keine explosionsfähigen Gasgemische vorliegen. Eine geeignete Zusammensetzung des Reaktionsgases zur Herstellung von Ethylenoxid kann z.B. aus ca. 30 Vol.-% Ethylen, ca. 7 Vol.-% Sauerstoff, 0,5 bis 5 ppm eines chlorhaltigen Reaktionsmoderators, wie Ethylchlorid, Vinylchlorid oder Dichlorethan, bestehen, wobei der Rest des Reaktionsgases in der Regel aus Kohlen-wasserstoffen, wie Methan oder Ethan, oder aber auch aus Inertgasen, wie Stickstoff, zusammengesetzt sein kann. Zusätzlich können auch noch andere Stoffe wie Wasserdampf, Kohlendioxid oder Edelgase im Reaktionsgas enthalten sein. Die Oxidation wird im allgemeinen bei Temperaturen von 165 bis 300 °C durchgeführt.

Vorteilhafterweise kann die Herstellung von Ethylenoxid aus Ethylen und Sauerstoff in einen Kreisprozess durchgeführt werden. Dabei wird das Reaktionsgasgemisch im Kreislauf durch den Reaktor geleitet und nach jedem Durchgang das neu gebildete Ethylenoxid sowie die bei der Umsetzung gebildeten Nebenprodukte aus dem Produktgasstrom entfernt, der nach Ergänzung mit den erforderlichen Mengen an Ethylen, Sauerstoff und Reaktionsmoderatoren wieder in den Reaktor zurückgeführt wird. Die Abtrennung des Ethylenoxids aus dem Produktgasstrom und seine Aufarbeitung können nach den üblichen Verfahren des Standes der Technik (vgl. Ullmann's Encyclopedia of Industrial Chemistry; 5th Ed.; Vol. A10; S. 117-135, 123-125; VCH Verlagsgesellschaft; Weinheim 1987) erfolgen.

Im Folgenden wird die vorliegende Erfindung anhand von Beispielen näher erläutert.

### BEISPIELE

Es wurden Katalysator A mit 8,6 Gew.% Silber und Katalysator B mit 18,6 Gew.% Silber eingesetzt.

### Zusammensetzung der Katalysatoren:

| Zusammensetzung: | Katalysator A | Katalysator B |
|---|---|---|
| Silber (Gew.%) | 8,6 | 18,6 |
| Lithium (Gew. ppm) | 190 | 190 |
| Schwefel (Gew. ppm) | 14 | 14 |
| Wolfram (Gew. ppm) | 200 | 200 |
| Cäsium (Gew. ppm) | 460 | 460 |
| Rhenium (Gew. ppm) | 310 | 310 |

Als Trägermaterial für beide Katalysatoren A und B wurde ein poröses Aluminiumoxid verwendet, das beispielsweise erhältlich ist von Süd-Chemie, Noritake, CeramTec oder Industriey Bitossi.

Die eingesetzten Katalysatoren A und B wurden nach üblichen Methoden hergestellt, wie beispielsweise in EP-A 266 015 offenbart.

Als Rohrreaktor wurde ein kreisringzylindrischer Doppelmantelreaktor aus Edelstahl vom DIN Typ 1.4541 verwendet. Der Innendurchmesser des durch den inneren Mantel begrenzten Rohres (des Reaktionsraums) betrug 6 mm. Die Wandstärke des inneren Mantels war 1 mm. Der Abstand zwischen dem äußeren und dem inneren Mantel lag bei 3,5 mm. Die Wandstärke des äußeren Mantels war 3,2 mm.

Als Temperiermedium wurde im zwischen den beiden Mänteln befindlichen Zwischenraum ein Wärmeträgeröl vom Typ AP 100 Silikonöl der Fa. Wacker Chemie AG, München, geführt (mit einer Umpumprate von ca. 15 l/min). Die Länge des Rohrreaktors betrug 2200 mm. Das Wärmeträgeröl wurde am unteren Ende des Rohrreaktors mit einer Eingangstemperatur T_{W}^{Ein} zugeführt und strömte von unten nach oben, wo es aus dem Mantelzwischenraum herausströmte.

Von unten nach oben war der Reaktionsraum wie folgt beschickt:
- eine Nachschüttung aus inerten Steatitkugeln (Steatit C220 der Fa. CeramTec) mit einem Kugeldurchmesser von 1,0 bis 1,6 mm in einer Schüttungsmenge von 9 g; die Schüttungslänge betrug ca. 21,2 cm;
- eine Schüttung aus 32 g Splitt der Katalysatoren mit einer Korngröße von 0,6 bis 0,9 mm; die Schüttungslänge betrug ca. 110 cm; und
- eine Vorschüttung aus inerten Steatitkugeln (Steatit C220 der Fa. CeramTec) mit einem Kugeldurchmesser von 1,0 bis 1,6 mm in einer Schüttungsmenge von 29 g; die Schüttungslänge betrug ca. 70,7 cm.

Auf seiner Restlänge war der Reaktionsraum leer.

Dem Reaktionsraum wurde von oben nach unten strömend ein Reaktionsgaseingangsgemisch zugeführt, das nachfolgende Zusammensetzung aufwies:
- 35 Vol.-% Ethylen,
- 7 Vol.-% molekularer Sauerstoff,
- 1 Vol.-% Kohlenstoffdioxid,
- 0,15 Vol.-% Wasser,
- 2 bis 6 Vol.ppm Ethylchlorid (Moderator) und
- als Restmenge bis zu 100 Vol.-% Methan.

Der Eingangsdruck des Reaktionsgasgemischs beim Eintritt in den Reaktionsraum betrug 16 bar absolut. Das Reaktionsgaseingangsgemisch war auf eine Temperatur von 130°C vorerwärmt. Die Belastung der ca. 110 cm langen Katalysatorsplittschüttung mit dem Reaktionsgaseingangsgemisch betrug 4750 h⁻¹. T_{W}^{Ein} wurde so gewählt und kontinuierlich angepasst, dass der Gehalt des Reaktionsgasgemischs beim Verlassen des Reaktionsraums stetig 2,7 Vol.-% Ethylenoxid betrug. Der Gehalt des Reaktionsgaseingangsgemischs an Ethylchlorid wurde über die Laufzeit so erhöht, dass zu jedem Betriebszeitpunk die maximale Selektivität der Ethylenoxidzielproduktbildung gewährleistet war.

Nach einer Einlaufphase von 300 Betriebsstunden wurden folgende Versuchsergebnisse erhalten:

| | |
|---|---|
| V1: 100 % Katalysator A: | T_{W}^{Ein} = 238,4°C; |
| | Ethylchloridgehalt: 3,0 Vol.ppm; |
| | S^{Eth} = 85,9 mol-%. |
| | |
| V2: 100 % Katalysator B: | T_{W}^{Ein} = 230, 9°°C; |
| | Ethylchloridgehalt: 2,6 Vol.ppm; |
| | S^{Eth} = 84,3 mol-%. |
| | |
| V3: 33 Gew.% Katalysator A (Schicht (i)) / 67 Gew.% Katalysator B (Schicht (ii)): | |
| | T_{W}^{Ein} = 232,6°C; |
| | Ethylchloridgehalt: 2,6 Vol.ppm; |
| | S^{Eth} = 85,3 mol-%. |

Es zeigte sich, dass ein Gewichtsverhältnis der Katalysatoren unterschiedlicher Silberzusammensetzungen von 1 (geringerer Silberanteil) : 2 (höherer Silberanteil) innerhalb der Katalysatorschüttung im Vergleich zum höher silberhaltigen Katalysator B zu einer nahezu identischen Aktivität und einer deutlich gesteigerten Selektivität führte.

## Patentansprüche

1. Verfahren zur Herstellung von Ethylenoxid durch die Umsetzung von Ethylen mit Sauerstoff in Gegenwart mindestens eines silberhaltigen Katalysators, wobei die Umsetzung in einem Reaktor stattfindet, der eine Katalysatorschüttung mit mindestens zwei Schichten (i) und (ii) aufweist, und der Silbergehalt des Katalysators in der Schicht (i) geringer ist als der Silbergehalt des Katalysators in der Schicht (ii), wobei das Reaktionsgemisch mit der Schicht (i) in Kontakt kommt, bevor es mit der Schicht (ii) in Kontakt kommt.

2. Verfahren nach Anspruch 1, wobei die Schicht (i) und die Schicht (ii) jeweils mindestens 10% der gesamten Katalysatorschüttung ausmachen.

3. Verfahren nach einem der Ansprüche 1 bis 2, wobei die Schicht (i) von 40 bis 70 % der gesamten Katalysatorschüttung ausmacht.

4. Verfahren nach einem der Ansprüche 1 bis 3, wobei die Schicht (ii) von 20 bis 60 % der gesamten Katalysatorschüttung ausmacht.

5. Verfahren nach einem der Ansprüche 1 bis 4, wobei die Katalysatorschüttung eine weitere Schicht (a) aufweist, mit der das Reaktionsgemisch vor den Schichten (i) und (ii) in Kontakt kommt, und der Silbergehalt des Katalysators in der Schicht (a) höher ist als der Silbergehalt des Katalysators in der Schicht (i).

6. Verfahren nach Anspruch 5, wobei die Schicht (a) höchstens 10% der gesamten Katalysatorschüttung ausmacht.

7. Verfahren nach einem der Ansprüche 1 bis 6, wobei der Gradient des Silbergehalts des Katalysators in den Schichten der Katalysatorschüttung über die gesamte Laufzeit des Verfahrens vorliegt.

8. Verfahren nach einem der Ansprüche 1 bis 7, wobei der Silbergehalt des Katalysators in der Schicht (i) um 5 bis 15 Gew.-% geringer ist als der Silbergehalt des Katalysators in der Schicht (ii).

9. Verfahren nach einem der Ansprüche 1 bis 8, wobei mindestens einer der in den Schichten eingesetzten Katalysatoren Rhenium enthält.

## Claims

1. A process for preparing ethylene oxide by reaction of ethylene with oxygen in the presence of at least one silver-comprising catalyst, wherein the reaction takes place in a reactor which has a catalyst bed having at least two zones (i) and (ii) and the silver content of the catalyst in zone (i) is lower than the silver content of the catalyst in zone (ii), wherein the reaction mixture comes into contact with zone (i) before it comes into contact with zone (ii).

2. The process according to claim 1, wherein zone (i) and zone (ii) each make up at least 10% of the total catalyst bed.

3. The process according to either claim 1 or 2, wherein zone (i) makes up from 40 to 70% of the total catalyst bed.

4. The process according to any of claims 1 to 3, wherein zone (ii) makes up from 20 to 60% of the total catalyst bed.

5. The process according to any of claims 1 to 4, wherein the catalyst bed has a further zone (a) with which the reaction mixture comes into contact before the zones (i) and (ii) and the silver content of the catalyst in the zone (a) is higher than the silver content of the catalyst in zone (i).

6. The process according to claim 5, wherein zone (a) makes up not more than 10% of the total catalyst bed.

7. The process according to any of claims 1 to 6, wherein the gradient of the silver content of the catalyst in the zones of the catalyst bed is present over the entire running time of the process.

8. The process according to any of claims 1 to 7, wherein the silver content of the catalyst in zone (i) is from 5 to 15% lower than the silver content of the catalyst in zone (ii).

9. The process according to any of claims 1 to 8, wherein at least one of the catalysts used in the zones comprises rhenium.

## Revendications

1. Procédé de fabrication d'oxyde d'éthylène par la mise en réaction d'éthylène avec de l'oxygène en présence d'au moins un catalyseur contenant de l'argent, dans lequel la réaction a lieu dans un réacteur qui comprend un garnissage catalytique comprenant au moins deux couches (i) et (ii), et la teneur en argent du catalyseur dans la couche (i) est inférieure à la teneur en argent du catalyseur dans la couche (ii), le mélange réactionnel entrant en contact avec la couche (i) avant d'entrer en contact avec la couche (ii).

2. Procédé selon la revendication 1, dans lequel la couche (i) et la couche (ii) représentent chacune au moins 10 % de la totalité du garnissage catalytique.

3. Procédé selon l'une quelconque des revendications 1 à 2, dans lequel la couche (i) représente 40 à 70 % de la totalité du garnissage catalytique.

4. Procédé selon l'une quelconque des revendications 1 à 3, dans lequel la couche (ii) représente 20 à 60 % de la totalité du garnissage catalytique.

5. Procédé selon l'une quelconque des revendications 1 à 4, dans lequel le garnissage catalytique comprend une couche (a) supplémentaire, avec laquelle le mélange réactionnel entre en contact avant les couches (i) et (ii), et la teneur en argent du catalyseur dans la couche (a) est supérieure à la teneur en argent du catalyseur dans la couche (i).

6. Procédé selon la revendication 5, dans lequel la couche (a) représente au plus 10 % de la totalité du garnissage catalytique.

7. Procédé selon l'une quelconque des revendications 1 à 6, dans lequel le gradient de la teneur en argent du catalyseur dans les couches du garnissage catalyseur est présent pendant la durée totale du procédé.

8. Procédé selon l'une quelconque des revendications 1 à 7, dans lequel la teneur en argent du catalyseur dans la couche (i) est inférieure de 5 à 15 % en poids à la teneur en argent du catalyseur dans la couche (ii).

9. Procédé selon l'une quelconque des revendications 1 à 8, dans lequel au moins un des catalyseurs utilisés dans les couches contient du rhénium.
